# EUROPEAN PATENT APPLICATION

(11) **EP 4 686 944 A1**
(43) Date of publication of application: **04.02.2026**
(21) Application number: 24192285.5
(22) Date of filing: 01.08.2024
(51) Int. Cl.: G01N 33/543, G01N 21/78, A61B 5/1473, G01N 21/00, A61B 5/00

(54) **SENSOR SKIN IMPLANT, METHOD FOR DETECTING THE PRESENCE OF AN ANALYTE WITH SUCH A SENSOR SKIN IMPLANT AND METHOD FOR MANUFACTURING OF SUCH A SENSOR SKIN IMPLANT**

(71) Applicant: Johannes Gutenberg-Universität Mainz, 55122 Mainz (DE)
(72) Inventor: SÖNNICHSEN, Carsten, 55124 Mainz (DE)
(74) Representative: Patentanwälte Dr. Keller, Schwertfeger Partnerschaft mbB

(57) **Abstract**

The invention relates to a sensor skin implant, a method for detecting the presence of an analyte with such a sensor skin implant and a method for manufacturing of such a sensor skin implant.

Implantable sensors can accompany medical treatments, easing the life of both physicians and patients by providing a comfortable way of continuously monitoring biomarkers without needing to take blood samples. For ideal sensor read-out and comparison of measurement results between patients, the individual implants must be as uniform as possible to exclude the influence of measurement artifacts caused by differences between implants. Additionally, the integration of the implant into the host tissue must be ensured for every single case to guarantee unrestricted analyte transport to the sensor and prevent non-working implants.

The object of the invention was in particular to find a new fabrication approach to produce nanoparticle-based macroporous implants enabling mass production with improved implant quality and a new method for detecting the presence of an analyte with such a sensor skin implant that shows a better or easier sensor read-out.

The invention solves this task particularly with a sensor skin implant comprising a plurality of alternating reference stripes and sensor stripes that are arranged next to each other to form a repetition unit, wherein the sensor stripes and references stripes are embedded in a bio-integrable macroporous hydrogel and are equipped with one type of nanoparticles, wherein the nanoparticles of the sensor stripes are coupled to or coated with a receptor bearing specific binding sites for an analyte.

## Description

The invention relates to a sensor skin implant, a method for detecting the presence of an analyte with such a sensor skin implant and a method for manufacturing of such a sensor skin implant.

Implantable sensors can accompany medical treatments, easing the life of both physicians and patients by providing a comfortable way of continuously monitoring biomarkers without needing to take blood samples. For ideal sensor read-out and comparison of measurement results between patients, the individual implants must be as uniform as possible to exclude the influence of measurement artifacts caused by differences between implants. Additionally, the integration of the implant into the host tissue must be ensured for every single case to guarantee unrestricted analyte transport to the sensor and prevent non-working implants. One approach is the integration of miniscule sensing elements into macroporous hydrogels.

Medical implantable sensors that continuously monitor biomarkers can accompany, support, and complement physicians treating patients. They significantly ease the life of patients with chronic diseases, e.g. diabetes, freeing them of the need to take regular blood samples. Approaches for such sensors are various, ranging from semi-implanted probes to wearable or implanted electrodes, electromagnetic implants and polymeric structures, often relying on dyes or enzymes for the actual sensing.

In Kaefer et al. (Kaefer, K.; Krüger, K.; Schlapp, F.; Uzun, H.; Celiksoy, S.; Flietel, B.; Heimann, A.; Schroeder, T.; Kempski, O.; Sönnichsen, C. Implantable Sensors Based on Gold Nanoparticles for Continuous Long-Term Concentration Monitoring in the Body. Nano letters 2021, 21 (7), 3325-3330. DOI: 10.1 021/acs.nanolett.1c00887. Published Online: Mar. 30, 2021), the feasibility of concentration-dependent measurements in living rats with a macroporous implant based on plasmonic nanosensors is shown.

Hereby, each implant was individually assembled in a time-consuming and laborious fabrication process, resulting in variances between implants and limiting advancements in both implant design and measurement procedure.

Against this background, it is the object of the present invention to overcome the aforementioned disadvantages of the prior art, in particular to find a new fabrication approach to produce nanoparticle-based macroporous implants enabling mass production with improved implant quality and a new method for detecting the presence of an analyte with such a sensor skin implant that shows a better or easier sensor read-out.

This object is solved by a sensor skin implant according to claim 1 and by a method for detecting the presence of an analyte with such a sensor skin implant according to the subsidiary claim 7 and a method for manufacturing such a sensor skin implant according to the subsidiary claim 13. Advantageous embodiments of the invention can be found in the dependent claims.

The above-mentioned inventions can be further improved or further characterized by the following features or advantageous embodiments, each of which is advantageous in itself and can be combined with one another as desired.

In a first aspect of the invention, it is a sensor skin implant comprising a plurality of alternating reference stripes and sensor stripes that are arranged next to each other to form a repetition unit, wherein the sensor stripes and references stripes are embedded in a bio-integrable macroporous hydrogel and are equipped with one type of nanoparticles, wherein the nanoparticles of the sensor stripes are coupled to or coated with a receptor bearing specific binding sites for an analyte.

A reference stripe as used in the context of the present invention refers to a stripe embedded in a bio-integrable macroporous hydrogel containing one type of nanoparticles.

A sensor stripe as used in the context of the present invention refers to a stripe embedded in a bio-integrable macroporous hydrogel containing the same type of nanoparticles as the reference stripe, whereby the nanoparticles of the sensor stripe are coupled to or coated with a receptor bearing specific binding sites for an analyte.

A repetition unit as used in the context of the present invention refers to one sensor and a neighboring reference stripe.

With this sensor skin implant, several responsive sensing parts are provided which can be used for measuring a signal in for instance a live rat upon analyte injection. This new design is expected to be groundbreaking for plasmonic implantable sensing, pathing the way for a simple and comfortable detection method, robust measurement results, automated evaluation, and a uniform production process.

This sensor skin implant enables further a novel technique of optical signal read-out using an attractive reflection-based setup where only at least-one wavelength, preferably only one wavelength, for detection is needed, which will be described in more detail in the following, and shows better tissue integration than implants shown in the state of the art, both resulting in a significantly improved and more robust signal measured in vivo. A robust signal refers to a signal that is not (or less) influenced by changes unrelated to the desired analyte, for example sweating, level of blood circulation or oxygenation, skin pigmentation, changes in the detection instrument, or similar issues.

In a preferred embodiment, the sensor skin implants and methods of the invention apply one wavelength for detection. One advantage of a monochromatic detection is that no additional filters or other cost-intensive parts are required. In alternative embodiments, the sensor skin implants and methods of the invention apply two wavelengths for detection. In these embodiments, two light sources, preferably two monochromatic LEDs or a broadband LED, can be utilized.

A further advantage of the sensor skin implants of the invention is that since the reference and sensor stripes in the sensor skin implant according to the invention are now in close spatial proximity, no additional second particle type is needed for correction against local changes. In an advantageous embodiment, the stripes alternate between thin (e.g. 1.6 mm) sensor and reference nanoparticle containing stripes. Preferably, the sensor skin implant shows alternatingly nine sensor and nine reference stripes with a total size of approximately 4 mm by approximately 27 mm. In preferred embodiments, the sensor stripes and/or the reference stripes each have a length in the range between 2.0 and 4.0 mm, and a width in the range between 1.0 and 2.0 mm, preferably a width of 1.6 mm.

In a further advantageous embodiment, the implantable sensor skin can be based on functionalized gold nanoparticles as sensors. Gold nanoparticles scatter and absorb light at a specific resonance wavelength that depends on their immediate environment, which makes them applicable as biosensors. For the here described sensor skin implant according to the invention, gold nanoparticles can be coated with an aptamer as receptor that specifically binds analytes, for instance the aminoglycoside kanamycin. Aptamers are single-stranded deoxyribonucleic acid (DNA) or ribonucleic acid (RNA) oligonucleotides, which are able to bind their target with high selectivity and affinity. As the plasmonic gold nanoparticles can be easily adaptable toward different analytes (as for example in the various lateral flow tests where antibodies detect, for example, COVID virus particles), this makes the sensor skin implant applicable to many personalized medicine applications.

Plasmonic nanoparticles change their resonance wavelength depending on their shape. Rod-shaped nanoparticles show a more red-shifted resonance wavelength compared to round particles. The invention here would work with any nanoparticle that shows a change in optical response in the presence of an analyte. In the preferred embodiment, gold nanorods are used, preferably with a diameter between 8 nm and 30 nm, and a length that is a multiple of the diameter with a factor of 2-5. The ratio between length and diameter, the aspect ratio, determines the resonance wavelength and is chosen such that it lies in the preferred spectral region that is determined, among other considerations, by the tissue window, the light source, and the detector.

In preferred embodiments, the sensor stripes are with plasmonic nanoparticles, preferably gold or gold alloy nanoparticles, that have a resonance wavelength in the near-infrared regime, preferably ranging from 700 nm to 3.000 nm, particularly preferably between 700 nm to 800 nm. A preferred wavelength region is between 700 nm and 900 nm, preferably around 800 nm, where skin is sufficiently transparent and optical elements are readily available.

Besides gold nanoparticles, other metal and magnetic nanoparticles, silica- or polymer-based nanoparticles and quantum dots can be used in alternative embodiments. For combined diagnostic and medical use, the silica- or polymer-based nanoparticles can be loaded with the receptor. They can additionally include organic dyes for optical detection, just as functional groups for their conjugation with aptamers.

The macroporous hydrogel can serve as a scaffold to fixate the sensor and reference stripes in one place while protecting them from degradation or cellular uptake. The macropores allow for vascularization, enabling a fast transport of the analyte to the nanosensors through the ingrown blood vessels and preventing implant encapsulation. Therefore, in an advantaged embodiment the macroporous hydrogel can have a pore size in the range between 10 and 50 µm.

In an advantageous embodiment of the sensor skin implant, as a bio-integrable macroporous hydrogel a copolymer of poly(2-hydroxyethyl methacrylate) and polyethylene glycole)diacrylate can be used, as these polymers work best as a matrix for the sensor skin implant according to the invention.

The aptamer-modified nanoparticles of the present invention are suitable for any medical application, preferably for the detection of analytes, for biomedical in-vivo imaging and for monitoring in-vivo drug delivering. In preferred embodiments, the plasmonic gold nanoparticles are coated with an aptamer as receptor that specifically binds to the analyte. Preferably the analyte is a biomarker indicative for the medical indication, preferably kanamycin, glucose, insulin, oxygen, or a cancer marker, or hormone, or inflammation marker, or any other small molecule of interest that is present in the blood.

Generally, implanted sensors are most useful if a continuous readout over an extended period is desirable, for example in chronic diseases. In principle, such sensors could also find applications in other areas such as monitoring of health parameters in professional or recreational sport. The strength of such an implanted sensor in comparison to a classical blood test is the potential for continuous readout, especially in a non-hospitalized setting (at home), which is most useful for chronic issues where the relevant blood parameters need to be monitored on a continuous level. An example would be a drug that is given to treat a chronic disease (e.g. HIV, arthrosis, depression, diabetes) where the drug level (or glucose level in the case of diabetes) in the blood needs to be controlled in a narrow window. Acute diseases such as cancer can also be addressed to monitor tumor parameters, for example certain blood values for prostate.

Furthermore, applications outside of monitoring diseases would also be conceivable where blood parameters need to be determined, such as parameters in samples obtained from athletes during training or competition, for fighter pilots or astronauts during flight training or mission, and divers. The test even allows the blood alcohol level to be determined in order to check a man or woman's fitness to drive.

In a second aspect of the invention, it is a method for detecting the presence of an analyte, comprising the steps:
a. providing a sensor skin implant according to the invention,
b. detecting the presence of analyte bound to the receptor by recording the reflection spectra at least one wavelength in the near-infrared regime ranging from 700 nm to 3.000 nm, preferably between 700 nm to 800 nm, wherein a binding of the analyte leads to a shift, in particular to a redshift, of the sensor stripes relative to the reference stripes, resulting in a contrast change between the sensor and reference stripes at the at least one of the (or the single) recording wavelength(s).

The sensor skin implant according to the invention allows therefore a new evaluation that does not require the recording of many wavelengths but only of at least one.

Preferably, light is emitted in the direction of the sensor skin implant by means of a light source, preferably a LED, with preferably monochromatic light, which is preferably in contact with the skin, under which the sensor skin implant is arranged in the near. Particularly preferably, the light source is thereby arranged lateral or orthogonal to the skin. One advantage hereby is that a minimum of reflection is achieved, whereby a sufficient amount of light reaches in the skin for the further examination. Another advantage lies in the usage of low-cost components, for instance by using LEDs as a light source. Furthermore, the usage of reflection and for these needed parts can enable a compact and simple design for a corresponding readout device, based on the principles shown by the invention.

Furthermore, in a preferred embodiment, the detection of the reflected light occurs with a detection device, which can be a camera, for instance a CMOS Sensor or a mobile phone camera, or any other device with which reflected light can be received in the near-infrared range. In an advantageous embodiment, if more than one detection wavelength is desired, the illumination is by a broadband (white) LED. In this case, the detection wavelength or multiple wavelengths of the reflected light can be chosen by passing it first through a filter that selects individual wavelengths (or small wavelength bands), preferably a liquid crystal tunable filter, whereby the light particularly preferably is focused on the detection device with an objective.

In a further advantageous embodiment, the detection of the presence of the analyte comprises the observation of changes in light absorption at the at least one wavelength over the implant's length. Preferably, detecting the presence of the analyte comprises the observation of changes in light absorption at a single wavelength over the implant's length. Preferably, detecting the presence of the analyte comprises the observation of changes in light absorption at not more than two wavelengths over the implant's length.

Furthermore, to improve the detection of the presence of the analyte in a further advantageous embodiment of the method for detecting the presence of an analyte only one wavelength is used at a known signal frequency as a sensing wavelength, and a second wavelength is optional for the correction of changes unrelated to the presence of the analyte. In an advantageous embodiment, one ideal wavelength for the evaluation is defined by considering the sensor particles' *λ*_{*r*es} and full width at half maximum *fwhm.* The effect of the absorbance increase on analyte binding in the sensor stripes is highest at or near the wavelengths, *λ*_{*r*es} + *fwhm*/*2,* which makes choosing this wavelength ideal for an embodiment with only one wavelength. This embodiment requires only a monochromatic light source (e.g. an LED) and no filters, and is therefore the most cost-efficient to implement in readout devices. In an embodiment that uses an existing camera, for example a smartphone, the only readout devices needed would be a cheap, monochromatic LED (or laser) coupled to a light delivery mechanism (glass fiber, prism or similar), and a mechanical holder for the camera or smartphone.

In a further advantageous embodiment, two ideal wavelengths for the evaluation are defined by considering the sensor particles' *λ*_{*r*es} and full width at half maximum *fwhm.* Since the effect of the absorbance increase is more significant at higher wavelengths, *λ*_{*r*es} + *fwhm*/*2* is used as the sensing wavelength and *λ*_{*r*es} - *fwhm*/*2* for correction, whereas λᵣₑₛ defines a specific resonance wavelength for the employed nanoparticles and *fwhm* defines the spectrum's full width at half maximum. The inventors found a maximal signal when evaluating only the sensing wavelength and a minimal signal when using the correction wavelength. As a result, combining both wavelengths makes the signal more robust against systematic errors and increases the signal-to-noise ratio. Preferably, the sensing wavelength ranges between 600 and 1.000 nm, particularly preferably 790 nm, and the optional correction wavelength ranges between 500 and 800 nm, particularly preferably 690 nm.

In a further advantageous embodiment, the detected changes are processed using a Fourier transformation, in particular a discrete cosine transformation, to identify and quantify the analyte based on modulation of the contrast at the spatial frequency corresponding to the spacing of sensor and reference stripes or a multiple thereof. This method works best in combination with a single detection wavelength, i.e., a monochromatic illumination.

In a further advantageous embodiment, the sensor is illuminated from the side of the implant, preferable with a light guide in contact with the skin, preferably from both sides of the implant, preferably with light emitted from an LED or laser.

In a further preferred embodiment, the reflected light emitted from the area of the skin with the implant is recorded with recording device, preferably selected from camera, line detector or photo-detector. In a preferred embodiment, the recording device comprises a camera and corresponding imaging optics. Reflected light means that it diffuses first into the skin, then interacts with the implant, then diffuses out.

As mentioned above, it is preferred that the illumination is monochromatic at a given time, i.e., if more than one wavelength is recorded, the illumination switches between different monochromatic light sources (LED, lasers).

In alternative embodiments, the illumination is broad ("white") and a filter in front of the recording camera is used to select one wavelength at a given time. If more than one wavelength is desired to be recorded, the filter changes over time.

It is also possible to record more than one wavelength by using two or more cameras simultaneously with filters or dichroic mirrors in front of them. Preferably, the camera is replaced with a line detector (one dimensional camera). Alternatively, the line detector is replaced by a single photo-detector that is moved across the implant area, either physically or by changing the optical path such that different places along the implant area are recorded at different times.

A last aspect of the invention relates to a method for manufacturing a sensor skin implant, in particular a sensor skin implant according to the invention, comprising the steps of:
- providing a bio-integrable macroporous hydrogel,
- embedding a plurality of alternating reference stripes and sensor stripes that are arranged next to each other to form a repetition unit, wherein the plurality of stripes is equipped with one type of nanoparticles, wherein the nanoparticles in the reference stripes are of a type that do not interact with the analyte, whereas the nanoparticles of the sensor stripes are coupled to or coated with a receptor bearing specific binding sites for an analyte.

An advantage of this method is that it enables the mass fabrication of uniform implants with better tissue integration than known from the prior art. The procedure is automatable, scalable, and flexibly adjustable to different designs while guaranteeing the equality of implants, which is especially useful for industrial upscaling the production with the necessary quality controls needed for a commercial product.

In a first advantageous embodiment of the method for manufacturing the sensor skin implant, the bio-integrable macroporous hydrogel with the plurality of alternating reference stripes and sensor stripes are fabricated by:
a. filling Polymethyl methacrylate (PMMA) beads in a suitable form and sintering the beads together to form a beadcake,
b. presoaking a first piece of beadcake in polymer solution and placing it in a polymerization mold,
c. filling up the polymerization mold with polymer solution to fully cover the beadcake,
d. stacking another presoaked beadcake on the first piece of beadcake,
e. repeating the steps a. to d. using polymer solution containing sensor and reference nanoparticles alternatingly to give a plurality of stocked beadcakes,
f. ablating the resulting block of stocked beadcakes to give access to the inner porous structure,
g. dissolving the beadcakes in Diclormethane (DCM) to give a macroporous polymer,
h. cutting the polymer into suitable pieces, giving multiple implants from the same block.

The fabrication process in the advantageous embodiment has several advantages against the known ones from the prior art:
1. The implant's stripes are homogeneous. The width of one stripe solely depends on the beadcake's thickness because of the horizontal stacking instead of the manual cut. Therefore, all stripes have nearly the same thickness (e.g. 1.56 ± 0.08 mm). Also, the stacking results in a comparable thickness of the different connecting strips (e.g. 0.09 ± 0.04 mm). Under the compression of the block's own weight, the strips become equally thick. Thus, the implant's stripes are very homogeneous and ideal for identifying the modulation of the sensor skin implant.
2. The stripe width is easily adjustable by changing the thickness of the beadcake's sinter mold allowing the thin stripe width necessary for the sensor skin implant. In other processes, the width relies on the manual cutting and handling of the cutting person.
3. The handling time per stripe is roughly 2 min, resulting in 36 min for a block of 18 stripes. Notably, the time depends on the number of stripes and not on the size of the block, allowing the production of even bigger blocks, and thus, more implants, with only minimal extra effort.
4. The fabrication of a polymer block and slicing after the assembly leads to the mass production of several implants from the same block. These implants are all comparable.
5. The manufacturing process according to the invention is automatable since it does not involve as many delicate manual steps as it is the case in the prior art. The process can be further simplified by automatically cutting all beadcakes uniformly, which would also facilitate the ablation process and reduce the waste on the edges caused by differently sized beadcakes as well.

The invention is described in further detail below with reference to a number of embodiments. In accordance with the above explanations, individual features present in the following embodiment examples can be omitted if the technical effect associated with this feature is not important. Conversely, a feature described above but not present in an embodiment example below can be added to the embodiment for instance if the technical effect associated with this feature is important for a particular application. The invention also covers combination of features of different embodiments resulting in individual embodiments that are not specifically described herein but derivable in the light of the present invention.

In summary, the methods of the invention can be used on humans in everyday setting, without the need of specialized equipment and skilled professionals that operate it - which would neglect many of the advantages of using an implanted sensor for blood analysis that doesn't require to take blood outside of the body. The main improvement to achieve this better and/or easier readout is a) to use a reflection-based readout, where the illumination light is administered by light guides close to the side of the implant, and a detection perpendicular to the implant, and b) to use just one (or possibly two) wavelength for illumination and detection, evaluating the sensor response not as a spectral shift but simply as a contrast change between sensor and reference stripes. These two improvements mean that the readout is fast, convenient, and simple, which makes it possible to fabricate a cheap and simple readout device that a patient can use at home.

It is shown in:
- Fig. 1: a schematic example of the skin sensor implant according to the invention and the principles of the associated measurement method;
- Fig. 2: results of the sensor skin implant according to the invention measured in vivo compared to a prior art implant design;
- Fig. 3: the schematic of the measurement in transmission according to the prior art and the measurement in reflection according to the invention;
- Fig. 4: the sensor skin implant according to the invention in reflection in vivo;
- Fig. 5: a real set-up of the measurement procedure according to Figure 3 by a living rat;
- Fig. 6: an example of the sequence of the manufacturing process according to the invention in a schematic representation;
- Fig. 7: real-color images of the manufacturing process for the sensor skin implant according to the invention;
- Fig. 8: hyperspectral images of the illumination with two opposing and one fiber on a human forearm;
- Fig. 9: a schematic overview of the in vitro measurement of the sensor skin implant according to the invention in a flow chamber; and
- Fig. 10: a real-color image of a flow chamber design with built-in sensor skin implant according to the invention.

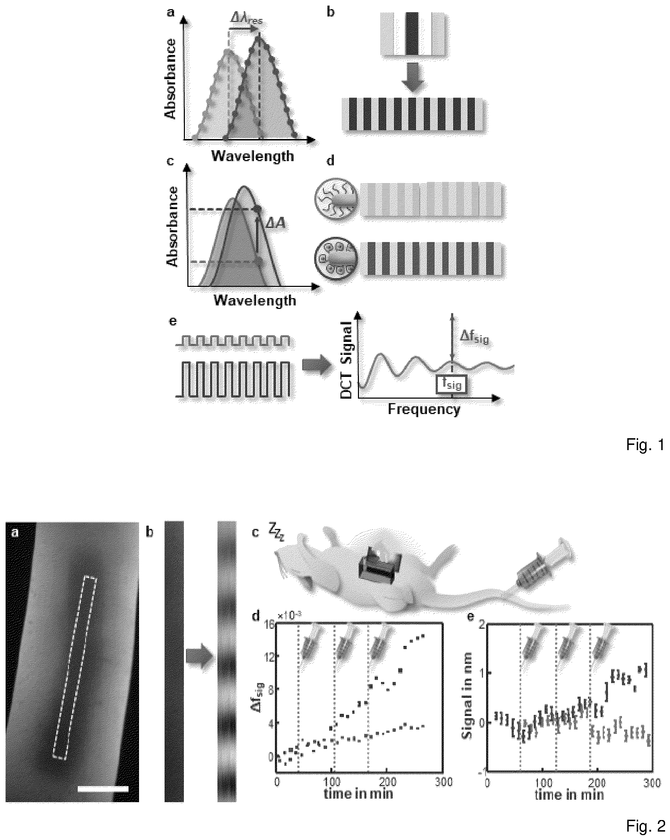

Figure 1 shows an example of the skin sensor implant and the principles of the associated measurement method. An implant design with a total of five stripes is known from the prior art (Figure 1, b, top). The two outer stripes contained non-responsive reference particles to correct for random changes during the measurement. The sensor particles were only located in the middle stripe. Two transparent stripes serve as a reference to calculate the spectrum of the nanoparticles and separate the three nanoparticle-containing stripes. A second type of non-responsive particles with a higher wavelength as an additional reference in each particle-containing stripe has been used to correct local changes because the reference and sensor stripe are far apart due to the stripe width of 3 mm. Pictures were taken at several wavelengths with a spectral imaging setup and the resonance wavelength *λ*_{*r*es} was determined with a gaussian function (Figure 1a). The final signal has been obtained after including the change of the resonance wavelengths *Δλᵣₑₛ* of the sensor and all the reference particles as described by Kaefer et al (Kaefer, K.; Krüger, K.; Schlapp, F.; Uzun, H.; Celiksoy, S.; Flietel, B.; Heimann, A.; Schroeder, T.; Kempski, O.; Sönnichsen, C. Implantable Sensors Based on Gold Nanoparticles for Continuous Long-Term Concentration Monitoring in the Body. Nano letters 2021, 21 (7), 3325-3330. DOI: 10.1021/acs.nanolett.1c00887. Published Online: Mar. 30, 2021).

The sensor skin implant according to the invention simplifies the implant fabrication, measurement, and data evaluation procedures. The sensor skin implant according to the invention solely has nanoparticle-containing stripes, and thus, no transparent ones that do not give analyte-related information. It is alternate between thin (1.6 mm) sensor and reference particle containing stripes in the fabrication of the sensor skin implant, shown in Figure 1, b, bottom. Since the reference and sensor stripes are now in close spatial proximity, no additional second particle type is needed for correction against local changes.

Figure 1a shows the principle of the input data required for the prior art determination. Many wavelengths have been recorded to determine the shift of the resonance wavelengths *Δλᵣₑₛ.* Figure 1b shows the schematic design of the prior art and the sensor skin implant according to the invention. In the prior art one (top), two types of nanoparticles were used. The implant consisted of 5 stripes: two reference stripes (light grey), one sensor stripe (dark grey), and two transparent stripes without particles (white). The sensor skin implant according to the invention (bottom) only needs one type of nanoparticle: Nine reference (light grey) and nine sensor (dark grey) stripes alternate. One sensor and the neighboring reference stripe form a repetition unit. Figure 1c shows the principle of the input data for the sensor skin implant according to the invention. Recording of only one wavelength is necessary to obtain the change in absorbance *ΔA.* Figure 1d shows the sketch of the sensor skin implant according to the invention reaction to the analyte. Without analyte, the stripes colors on a grey scale are nearly identical (top). Upon analyte binding, the sensor stripes show an increase in absorbance, whereas the reference stripes show no such change. This results in a darker color for the sensor stripes (bottom). Figure 1e shows the principle of the signal detection for the sensor skin implant. Following the mean grey value (Figure 1d) along the sensor skin implant length, only little modulation without analyte (Figure 1e, left top) and an increased modulation with analyte (Figure 1e, left bottom) is shown. Applying a discrete cosine transformation (DCT) to this signal and following the amplitude change at the repetition unit's frequency (signal frequency *f_{sig}*) gives the signal *Δf_{sig}.*

The sensor skin implant according to the invention allows therefore a new evaluation that does not require the recording of many wavelengths but only at least of one. Following the absorbance change of the spectrum at a wavelength higher than *λ*_{*r*es} (at a preferably sensing wavelength *λ*ₛₑₙₛ of 790 nm, near *λ*_{*r*es} + *fwhm*/*2,* where fwhm stands for the width of the plasmon resonance peak at half maximum) results in an increase caused by a shift of *λ*_{*r*es} to higher wavelengths in the presence of an analyte.

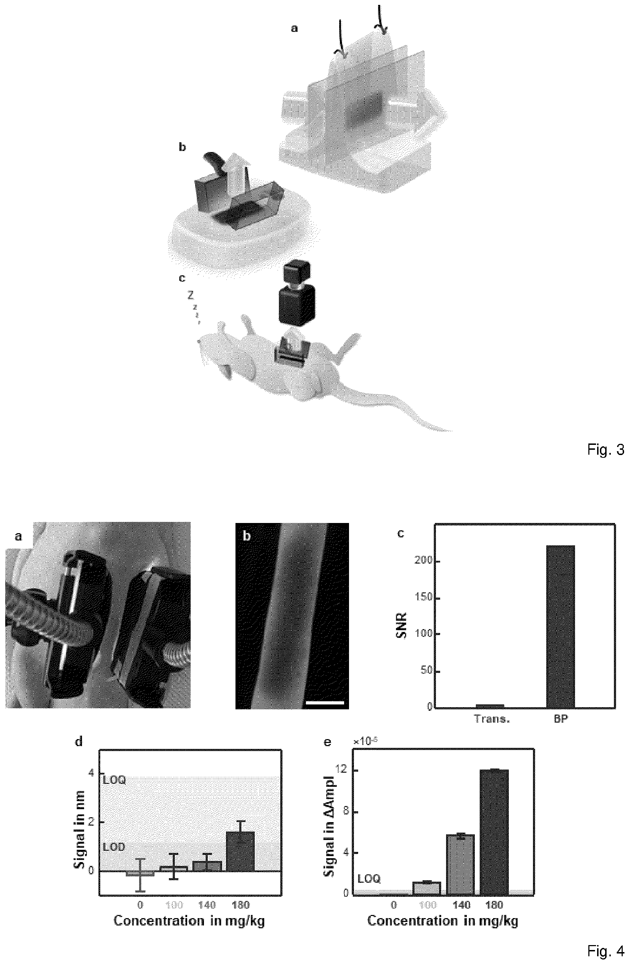

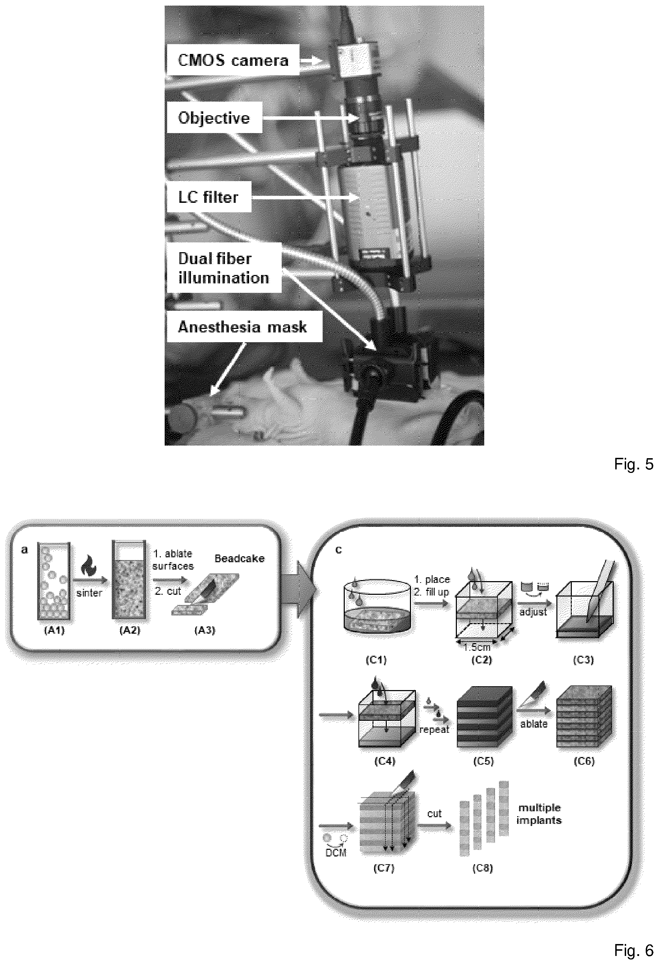

In the presence of an analyte, here kanamycin, the absorbance of the sensor stripes increases (i.e., the stripes darken) while the reference stripes stay unaffected (see Figure 1d, bottom). Consequently, instead of *Δλᵣₑₛ,* we compare the grey values of pictures of the sensor skin implant obtained with the setup's CMOS camera (see Figure 3c and Figure 5) at the sensing wavelength *λ*ₛₑₙₛ (see Figure 3c). Comparing the grey value changes of a sensor stripe to its neighboring reference stripe gives information on the binding of an analyte, here kanamycin. Therefore, two neighboring reference/sensor stripes are here called a sensing couple with a length of 3.2 mm.

For data evaluation, the mean grey value of the stripes is used, giving a line profile over the sensor skin implant length. This profile shows only little modulation without kanamycin (Figure 1e, left top) due to the similar absorbance of the reference and sensor stripes. The binding of an analyte, here kanamycin, results in a darkening of the sensor stripes, and, thus, a modulation increase (Figure 1e, left bottom).

In an advantaged embodiment, a Fourier transformation, here especially a discrete cosine transformation (DCT) to this modulated signal is applied and the change of the amplitude at the sensing couple's frequency, the signal frequency *f_{sig}* (0.31 mm⁻¹), is monitored since this is the sensor skin implant's smallest repetition unit (Figure 1e, right). The amplitude change at the signal frequency is here defined as the signal *Δf_{sig}.* In principle, this procedure could be repeated at different light frequencies, to obtain a spectral reference, for example by using the lower wavelength side of the resonance peak, where analyte binding has little effect. However, the signal at just one wavelength is already good enough for most applications, so the additional complexity of changing wavelength (filters, broadband illumination, or more than one light source) needs to be weighted in light of cost-effectiveness.

The sensor skin implant used here has alternatingly nine sensor and nine reference stripes with a size of approximately 4 x 27 mm. While those stripes are macroporous, they are connected here by thin non-porous lines caused by the here used fabrication process according to the invention. These thin non-porous lines appear dark in the images but are not visible once implanted due to the diffuse scattering effect of the skin.

To make the measurement much easier and more comfortable, a reflection-based setup was introduced by the inventors. Here in this example, two-line fibers are placed adjacent to the implant for indirect illumination with white light. The reflected light passes through a filter that selects individual wavelengths recorded by a CMOS camera (see Figure 3b, 3c, Figure 4a and Figure 5).

Figure 2 shows the implantation of the sensor skin implant in the abdominal skin of a hairless rat and the measurement of it with a reflection-based setup as described in Figure 3b and c. The implant is clearly visible as shown in the hyperspectral image at 790 nm (Figure 2a). A region of interest (ROI) has been chosen inside the sensor skin implant for further evaluation, unfortunately, the ROI has to be smaller than the sensor due to overexposure at the implant's edges, resulting in the loss of some of the sensor skin implant stripes. The image in the ROI shows no stripes due to the worse spatial resolution and the noise added by the skin (Figure 2b, left). When applying a bandpass filter at *f_{sig}* to the ROI, the stripes become clearly visible, with dark and light parts showing sensor and reference stripes respectively (Figures 2b, right).

Figure 2a shows a hyperspectral image of a sensor skin implant implanted in a live rat and recorded with a reflection-based setup at 790 nm. The white dotted box indicates the region of interest (ROI) used for evaluation. The scalebar is 5mm. Figure 2b shows the ROI used for evaluation as shown in Figure 2b, left and the same ROI after using a bandpass filtering at the signal frequency (right). The dark bars indicate the reference and the light bars the sensor stripes of the sensor skin implant. Figure 2c shows a scheme of the sensor skin implant measurement in vivo with kanamycin injection. The rat lies on its back, the sensor skin implant implanted in the abdominal skin is measured as described in Figure 3b and 3c. Kanamycin is injected at three different times in escalating dosages via the tail vein. Figure 2d shows *Δf_{sig}* of the implanted sensor skin implant over time. The lines represent the mean and the standard error of the mean over 10 minutes. The upper data set shows *Δf_{sig}* upon injection of kanamycin at three different times in escalating dosages. The lower data set corresponds to a control experiment with an injection of saline instead. The vertical dotted grey lines indicate the administration of kanamycin or saline solution. Figure 2e shows the signal of the prior art implant design by Kaefer et al (Kaefer, K.; Krüger, K.; Schlapp, F.; Uzun, H.; Celiksoy, S.; Flietel, B.; Heimann, A.; Schroeder, T.; Kempski, O.; Sönnichsen, C. Implantable Sensors Based on Gold Nanoparticles for Continuous Long-Term Concentration Monitoring in the Body. Nano letters 2021, 21 (7), 3325-3330. DOI: 10.1021/acs.nanolett.1c00887. Published Online: Mar. 30, 2021) measured in a transmission-based setup. The vertical dots are the mean and the lines are the corresponding standard error of the mean over 10 minutes. The upper data set shows the signal change in nm upon injection of kanamycin at three different times in escalating dosages. The lower data set is a control experiment with an injection of saline. The vertical dotted grey lines indicate the administration of kanamycin or saline solution. The error bars are significantly higher than in Figure 2d.

Kanamycin was injected via the rat's tail vein in three escalating dosages after the recording of a baseline (Figure 4c). The ROI was evaluated, *Δf_{sig}* was observed over time, and an increase was seen after the injections of kanamycin as expected. Repeating the same measurement with the injection of saline solution without kanamycin resulted in no such strong increase of *Δf_{sig}* (Figure 2d). Monitoring *Δf_{sig}* at 671 nm shows that the small increase is visible in both wavelengths, indicating a systematic error causing the increase. It is observed a signal increase upon injection of kanamycin and a random fluctuation when injecting saline solution instead (Figures 4e). However, the error bars are much bigger, impairing the signal-to-noise ratio drastically compared to the evaluation of the sensor skin implant according to the invention.

It is notable that the signal increase with the sensor skin implant according to the invention is much faster than with an implant according to Kaefer et al (Kaefer, K.; Krüger, K.; Schlapp, F.; Uzun, H.; Celiksoy, S.; Flietel, B.; Heimann, A.; Schroeder, T.; Kempski, O.; Sönnichsen, C. Implantable Sensors Based on Gold Nanoparticles for Continuous Long-Term Concentration Monitoring in the Body. Nano letters 2021, 21 (7), 3325-3330. DOI: 10.1021/acs.nanolett.1c00887. Published Online: Mar. 30, 2021). It is assumed that this is caused by the improved blood circulation at the sensor skin implant that is possible with the reflection-based setup according to the invention.

Figure 3 shows the schematic of the measurement in transmission according to the prior art and the measurement in reflection according to the invention. Figure 3a shows a scheme of a transmission measurement according to the prior art in vivo. The skin around the implant (black) is lifted to form a skin fold with the erected implant and surgical threads fix the fold in an upright position. Two microscope objective slides are pressed against the skin from both sides to smoothen the surfaces. In this setup, light enters the skin fold on one side and is detected on the other. Even though, if it is carefully paid attention to minimize pressure, the fold deviates from the natural position of the skin, possibly affecting blood circulation.

Therefore, the inventors aimed at a reflection-based illumination as a pressure-free, easy and comfortable alternative (Figure 3b). Figure 3b shows the principle of the reflection setup according to the invention. Two-line fibers transmit white light directly into the skin adjacent to the implant. Via diffuse scattering, the light passes the implant and emits through the skin. After selecting individual wavelengths with a liquid crystal tunable filter (LC filter) (see also Figure 5), the light is focused with an objective on a CMOS camera, taking images of the implant.

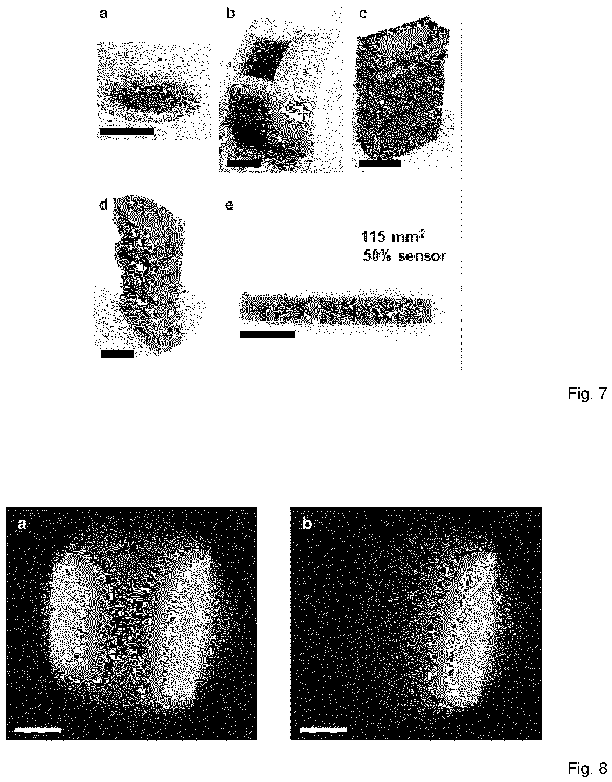

For the indirect illumination in the reflection setup, line fibers are brought in direct contact to the skin and a halogen lamp is used as light source. Figure 8 shows hyperspectral images of the illumination with two opposing and one fiber on a human forearm. Very close to the fiber the images are overexposed due to direct reflection of the light on the skin's surface. The corresponding illumination profiles in Figure 8b show the parabolic illumination when using two fibers and the exponential decease of one fiber. Figure 8a shows that with two fibers an area with homogeneous illumination is in the center of the fibers. Figure 8b shows that the light intensity decays exponentially when using only one fiber for illumination. The scale bars in Figure 8 are 10 mm.

For the setup shown in Figure 5 the rat's abdominal skin was cleaned with ethanolic skin disinfectant and then two dual line fibers (Thorlabs, OSL2FB-LINE-SP) were placed next to the implant and fixed with flexible gooseneck holders. A white light halogen lamp (Thorlabs OSL2) was used as the light source to illuminate the skin through the line fibers. The lamp's output was adjusted to prevent overexposure and overheating of skin, and thus, potentially burning. A LC filter (Thorlabs, KURIOS-XE2) mounted over the implant allowed selection of individual wavelengths. For measurement of the sensor skin implant according to the invention, 25 wavelengths between 650 and 818 nm were selected with a step size of 7 nm but only two wavelengths were used afterward for the evaluation. The light leaving the LC filter was focused via an objective (Edmund Optics, VIS-NIR fixed focal length) on a CMOS camera (Thorlabs, DCC3240N) recording all images. Black cloth was used to shield the setup from ambient light.

As only 4-7 % of incident light is directly reflected at the skin's surface, meaning that 93-96 % of the light enters the skin and is either elastically scattered due to inhomogeneities in the tissue's reflective index, e.g., collagen fibers, or absorbed by chromophores, e.g., melatonin. To minimize light loss due to absorption, gold nanoparticles with a resonance wavelength in the tissue window are used, where absorption is minimal. Due to the highly inhomogeneous composition of tissue, the light is scattered multiple times, resulting in a diffusive-like behavior in tissue with propagation in all directions and even remittance back through the skin, making the implant visible with an indirect illumination as described in Figures 3b and 3c.

Figure 4a shows a real color image of the anesthetized rat with the dual-fiber illumination of the sensor skin implant according to the invention. The implant is silhouetted dark against the bright skin, indicating the high absorbance due to the plasmonic nanoparticles. No individual stripes are identifiable in the sensor skin implant (see Figure 4b) because it lacks transparent stripes and both the reference and sensor stripes appear dark. Notably, the sensor skin implant is thinner than 5 mm, fitting ideally in the minimum of the parabolic illumination profile. The scale bar is 5 mm.

At the beginning of the measurement, a caudal vein catheter was placed and connected via tubing to a 3-way valve to inject a sterile 50 mg/mL kanamycin solution in a 0.9 % saline solution. One valve outlet was used to connect a syringe for bolus injections, the other outlet served for continuous infusion with a syringe pump.

While recording the baseline time trace, the syringe pump was used to inject the kanamycin solution at 50 µL/h to avoid catheter closure through agglomerated blood. The first kanamycin dose of 100 mg/kg was injected via the bolus syringe over 3-5 min. After 1 h, the second dose of 40 mg/kg was administered within 3 min, and an additional hour later, the third dose of 40 mg/kg was injected within 3 min. After each bolus injection, the syringe pump was used to continuously administer kanamycin solution to counter kanamycin degradation and excretion. The rates were 20 mg/kg/h, 28 mg/kg/h, and 36 mg/kg/h.

Figure 4c shows the signal-to-noise ratio of the in vivo measurements in transmission with the prior art 5-stripe-implant according to Kaefer et al. (Kaefer, K.; Krüger, K.; Schlapp, F.; Uzun, H.; Celiksoy, S.; Flietel, B.; Heimann, A.; Schroeder, T.; Kempski, O.; Sönnichsen, C. Implantable Sensors Based on Gold Nanoparticles for Continuous Long-Term Concentration Monitoring in the Body. Nano letters 2021, 21 (7), 3325-3330. DOI: 10.1021/acs.nanolett.1c00887. Published Online: Mar. 30, 2021) (Figure 4c, left bar) and in reflection with the sensor skin implant (Figure 4c, right bar).

Figure 4d shows the signal size for the transmission measurement in vivo with the prior-art 5-stripe implant design. LOD stands for limit of detection (see Figure 4d) and LOQ stands for limit of quantification (see Figure 4d and 4e). The bar depicts the signal size at the end of the baseline and the lines indicate the respective standard error. The bars are the signal size at the end of each concentration measurement in escalating order and the lines show the corresponding standard error.

Figure 4e shows the signal size for the reflection measurement in vivo with the sensor skin implant. The LOD is not visible since it is too small. The bars show the signal size at the end of the baseline and three different concentrations. The lines are the standard error of the mean. The bar indicating the signal size at the end of the baseline is too small to be visible.

The implants were measured over the course of several hours while injecting the analyte, kanamycin, via a teil vein catheter to test the functionality of the prior-art 5-stripe implant and the sensor skin implant according to the invention in live rats. Since the recorded baseline was noisier in reflection, the 5-stripe implant was measured only in transmission. For the measurement of the sensor skin implant, the reflection setup according to the invention was used. A baseline without analyte before administering kanamycin was recorded in three escalating dosages.

The corresponding time traces show a significant increase in the signal upon injection of kanamycin. While the prior-art 5-stripe implant exhibits large error bars and a delayed signal increase, the sensor skin implant according to the invention time trace has smaller error bars and rises fast after the first kanamycin injection. It is believed from the inventors that the time delay in the prior-art 5-stripe implant is caused by the hindered blood circulation caused by the artificial skin fold necessary for the transmission setup. In the sensor skin implant reflection measurement, the implant is pressure-free in a natural position, allowing good blood circulation, and thus, fast analyte transport into the implant. To verify that the signal is caused by the binding of kanamycin, the measurement was repeated but administered an analyte-free saline solution as a control. As expected, the corresponding time traces do not show a signal increase but fluctuate around the baseline. Notably, the control measurement of the sensor skin implant according to the invention is much less fluctuating than the prior-art one of the 5-stripe implant.

Thus, the inventors have developed a reflection-based setup for non-invasive measurement of plasmonic implants with indirect illumination in living rats at different wavelengths. In combination with the sensor skin implant according to the invention, the influence of the animal's respiratory movement on the measurement could also be overcome by using the sensor skin implant according to the invention, as only one wavelength is required for evaluation.

With the here described method for detecting the presence of an analyte with a sensor skin implant according to the invention, it can be seen a faster signal increase, stating the enhanced blood circulation. Together with the sensor skin implant according to the invention, it can be obtained a much-improved signal-to-noise ratio, needing only at least one, preferably two wavelengths for the measurement. The here described inventive reflection setup is at the crossroad of implementing medical plasmon-based implantable sensors. The setup can be simplified further by using LEDs, minimizing its size to create a handheld version for easy usage, thus, making it attractive for application in human patients as well.

Figure 6 shows an example of the sequence of the manufacturing process according to the invention in a schematic representation, whereby Figure 7 shows a real-color image of the manufacturing process for the sensor skin implant according to the invention.

To create macroporous implants, the sacrificial template method is used and a mold of sintered poly(methyl methacrylate) (PMMA) bead has been prepared (Figure 6a, A1). After sintering (Figure 6a, A2), the outer surfaces are smoothly molten due to contact with the form and must be ablated carefully to open access to the inner porous structure. Here, this porous PMMA structure is called the beadcake and it serves as a negative mold for the implant's hydrogel scaffold. Dissolution of the beadcake leaves behind macropores in the hydrogel, which are necessary for implant integration into the host tissue. The beadcake's thickness can be varied by adjusting the dimensions of the sinter form. Before using the beadcake in further processes, it is cut into pieces of the desired size (Figure 6a, A3).

Figure 6c shows the manufacturing process of the sensor skin implant according to the invention in a schematic representation. One piece of beadcake is presoaked in the polymer solution (Figure 6c, C1) and placed in the polymerization mold. The polymer solution is added again for full coverage of the beadcake (Figure 6c, C2). Excess of solution is removed with a pipette from the beadcake's top (Figure 6c, C3). Another presoaked beadcake is stacked on the first one (Figure 6c, C4). Steps C1-C3 are repeated using polymer solutions containing sensor and reference particles alternatingly to yield overall 18 stacked beadcakes (Figure 6c, C5). All surfaces of the resulting block are ablated to give access to the inner porous structure (C6). After dissolving the beadcakes in DCM only the macroporous polymer remains (Figure 6c, C7). Cutting the polymer into suitable pieces gives multiple implants from the same block (Figure 6c, C8).

The production process of the sensor skin implant according to the invention meets all necessary needs. Instead of cutting the beadcakes to the most fitting height as done in the prior art, the thickness of the sinter form has been increased to 1.5 mm, using a square polymerization mold with an edge length of 15 mm, and the beadcakes were stacked horizontally to achieve a uniform stripe height of 1.5 mm for the whole implant.

Each beadcake has been presoaked in the suitable polymer solution, placed in the polymerization mold, and excess of polymer solution was removed with a pipette, circumventing the vacuum-pressure cycle known from the prior art. By repeating these steps, with polymer solution containing sensor and reference particles alternatingly, a big block with overall 18 layers of beadcakes could be obtained. After the polymerization was finished, all surfaces with a scalpel were ablated and the beadcakes were dissolved in dichloromethane, leaving behind the macroporous hydrogel structure. Finally, the macroporous hydrogel block has been cut into pieces (which results in solely open surfaces) to obtain multiple equivalent implants.

Figure 7 shows real-color images of the different steps in the production process of the sensor skin implant according to the invention. The inventors exploit that the dry beadcake is highly liquid absorbing by the presoaking in the corresponding polymer solution (Figure 7a).

Figure 7b shows the silicon polymerization mold after the assembly of the polymer block. Its size has been halved to 15 x 7 mm in this fabrication example. The silicon ensures the safe removal of the sensitive block since the polymer does not adhere, is easily cleanable, and reusable.

After the polymerization is finished, the polymer block still contains all the beadcakes and its surfaces are sealed by non-porous polymer (Figure 7c). A scalpel is used to carefully ablate all surfaces and open access to the inner porous system and facilitate the diffusion into the block before dissolving the beadcakes in Dichlormethane in the next step. After the dissolution, only the macroporous hydrogel containing the nanoparticles remains (Figure 7d). Its surfaces are less straight than the ones of the block shown in Figure 7c because of the ablation process revealing slight differences in the beadcakes' sizes.

A razor blade was used to cut thin slices from the macroporous block and straighten the edges to obtain a ready-to-use sensor skin implant according to the invention (Figure 7e). Assuming a thickness of 1 mm, and a waste of 1 mm on each of the block's sides, 13 equivalent implants of 4.5 x 35 mm could be obtained from one block. If the full size of the polymerization mold (15 x 15 mm) would be used up to 36 equivalent implants at once could be fabricated. Notably, the fabrication time would however not increase with expanding the mold since it only depends on the number of stripes.

The gold nanoparticles used here in the manufacturing process were prepared as follows: First, a seed solution was prepared by adding 5 mL 0.2 M CTAB to a 0.001 M HAuCl₄ solution in water. 600 µL of a freshly prepared 20 mM solution of NaBH₃ in ice cold water were added under vigorous stirring. The mixture was left undisturbed for 1 h at room temperature and was used immediately after the incubation.

Second, a growth solution was prepared by dissolving 9 g CTAB and 1 g Br-SA in 250 mL water and heated to 37 °C. 11 mL of 4 mM AgNOs solution in water were added and the mixture was kept undisturbed at room temperature for 15 min. 2 mL of ascorbic acid were added and the mixture was stirred until the solution became colorless. Afterward, 200 µL of the seed solution was added the mixture was stirred vigorously for 30 s. Finally, the solution was kept undisturbed for 12 h at 37 °C.

To remove excess of growth solution, the solution was centrifuged at 6430 rcf for 20 min in 45 mL portions. The supernatant was removed, all pellets were combined and redispersed in 0.1 M CTAB solution in water.

To achieve sensor particles, the following steps have been carried out:
First, a reaction mixture of 1660 µL of Tris buffer, 100 µL of 100 µM Aptamer solution in Tris buffer with 100 µL of a 1 wt% SDS solution in water and 20 µL of TCEP solution (100 mM in a mixture of water:1M NaOH, 2:1) was prepared and incubated for 30 min. Second, 125 µl of gold nanorods were mixed with 375 µL 60 mM CTAB solution and centrifuged at 8070 rcf for 5 min. The supernatant was removed and the pellet was added to the reaction mixture. The resulting mixture was incubated for 3 h and inverted in between several times to prevent sedimenting. Then, the mixture was centrifuged at 6430 rcf for 7 min and the supernatant was removed.

The reference particles used here in the manufacturing process were prepared as follows: To create the reaction mixture, a 450 µL of 10 mM PEG solution in water was mixed with 50µL of a 2 % (v/v) TWEEN solution in water. 500 µL of gold nanorods were centrifuged at 9050 rcf for 6 min. The supernatant was removed and the pellet was added to the PEG solution. The mixture was incubated for 1 h. Afterwards, 2 M KCI solution was added in portions of 100 µL followed by an incubation of 10 min. In total, 500 µL KCI solution were added followed by a short incubation. Finally, the mixture was centrifuged at 8070 rcf for 7 min and the supernatant was removed.

Measuring the plasmonic spectrum of gold nanoparticles embedded in macroporous hydrogels is not trivial due to the fragile nature of the hydrogel, the high light scattering caused by the macropores, and the quick dehydrating. In the past, the nanoparticles' behavior in the hydrogel were mostly characterized by producing massive gels without macropores, recording spectra in an Ocean Optics spectrometer, and assuming a similar behavior in the macroporous hydrogels. The biggest drawback of this procedure was that it could not be measured with the hyperspectral transmission setup, which is normally used for in vivo measurements. This was especially problematic for the sensor skin implant according to the invention, since a full image for intensity-based analysis is needed.

To overcome this problem, a flow chamber was designed for in vitro analysis and defined several requirements it must fulfill: 1. Liquids must be exchangeable during the measurement to allow injection of analyte. 2. The flow chamber must fit into the hyperspectral transmission setup. 3. The chamber must fix the hydrogel in one place and prevent movement in the liquid stream. 4. The hydrogel must be completely immersed in the liquid from all sides to ensure fast diffusion of analyte to the nanosensors. 5. The design must be flexible to adjust for different implant sizes. 6. The flow chamber must be leakage proof.

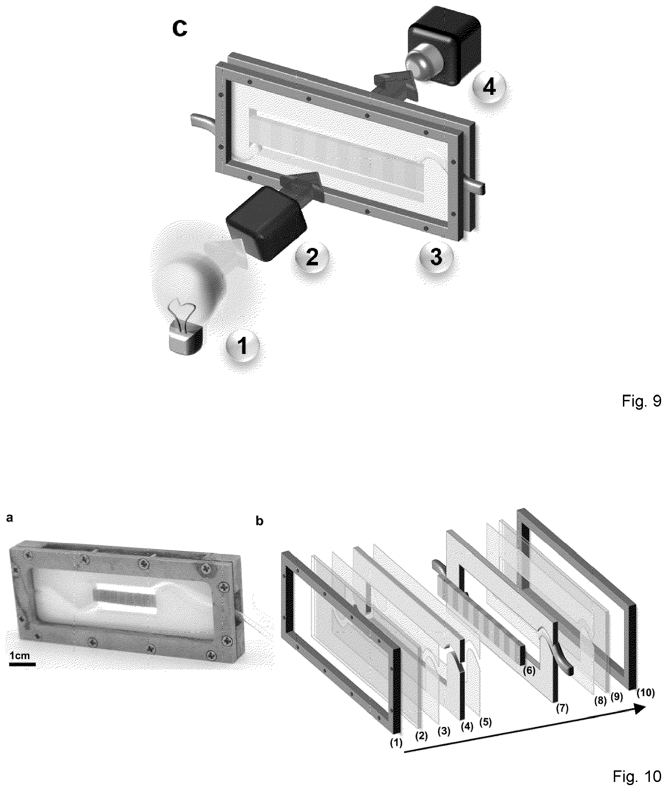

Figure 9 shows an overview of the schematic of an in vitro measurement of a sensor skin implant according to the invention with a flow chamber. In the transmission setup white light (Figure 9, (1)) passes a liquid crystal tunable filter (Figure 9, (2)), allowing only individual wavelengths to illuminate the sensor skin implant according to the invention in the flow chamber (Figure 9, (3)). The transmitted light is focused through an objective on a CMOS camera (Figure 9, (4)).

Figure 10a shows a real-color image of the final flow chamber design with built-in sensor skin implant according to the invention that fulfills all above mentioned requirements. The sensor skin implant is safely hold in place by the spacers (Figure 10b, (4) and (7)) while it is fully immersed in the surrounding liquid from all sides.

Figure 10b shows a scheme of the assembly. Glass slides (Figure 10b, (2) and (9)) confine the liquid containing chamber. The centerpieces of the flow chamber are 3D-printed thermoplastic polyurethane (TPU) spacer (Figure 10b, (4) and (7)) that fix the sensor skin implant according to the invention in one place. Because they are newly printed for each flow chamber, they are easily adjustable to different implant sizes and thicknesses. They are designed to fix the sensor skin implant according to the invention only at the edges and give enough space to ensure complete immersion of the sensor skin implant according to the invention from all sides. Additionally, they leave room for tubing that are connected with a three-way-valve and a syringe pump during the measurement to enable constant flow and easy exchange of liquid. Glass slides and spacer are closely attached with dual-sided adhesive foil (Figure 10b, (3), (5) and (8)), which enable a clear view on the sensor skin implant according to the invention. Tubing (Figure 10b, in (7)) allows flooding the chamber with liquid. Three Sheets of dual-sided adhesive foil cut in the shape of the TPU spacer stick together the spacer and objective slides to avoid leakage between the layers. Two brass frames (Figure 10b, (1) and (10)) with screws hold all parts tightly together. After the assembly, the tubes are fastened with UV glue and all sides are sealed with clear coat.

In summary, the invention provides a skin sensor implant, a method for detecting the presence of an analyte with such a sensor skin implant and a method for manufacturing of such a sensor skin implant, providing a new fabrication approach to produce nanoparticle-based macroporous implants enabling mass production with improved implant quality and a new method for detecting the presence of an analyte with such a sensor skin implant that shows a better or easier sensor read-out.

## Claims

1. A sensor skin implant, comprising a plurality of alternating reference stripes and sensor stripes that are arranged next to each other to form a repetition unit, wherein the sensor stripes and references stripes comprise a bio-integrable macroporous hydrogel and are equipped with one type of nanoparticles, wherein the nanoparticles of the sensor stripes are coupled to or coated with a receptor bearing specific binding sites for an analyte.

2. The sensor skin implant according to claim 1 or claim 2, wherein the bio-integrable macroporous hydrogel comprises a copolymer of poly(2-hydroxyethyl methacrylate) and polyethylene glycole)diacrylate.

3. The sensor skin implant according to any one of claims 1 to 2, wherein the sensor stripes and/or the reference stripes have each a length in the range between 2.0 and 4.0 mm, and a width in the range between 1.0 and 2.0 mm, preferably a width of 1.6 mm.

4. The sensor skin implant according to any one of claims 1 to 3, wherein the sensor stripes are equipped with plasmonic nanoparticles, preferably gold or gold alloy nanoparticles, that have a resonance wavelength in the near-infrared regime, preferably ranging from 700 nm to 3.000 nm, particularly preferably between 700 nm to 800 nm.

5. The sensor skin implant according to claim 4, wherein the plasmonic gold nanoparticles are coated with an aptamer as receptor that specifically binds to the analyte, wherein the analyte is a biomarker indicative for the medical indication, preferably kanamycin, glucose, insulin, oxygen, or a cancer marker, or hormone, or inflammation marker.

6. A method for detecting the presence of an analyte, comprising the steps
a. provision of a sensor skin implant according to any one of the claims 1 to 5,
b. detecting the presence of analyte bound to the receptor by recording the reflection spectra at least one wavelength in the near-infrared regime, preferably ranging from 700 nm to 3.000 nm, particularly preferably between 700 nm to 800 nm, wherein a binding of the analyte leads to a shift, in particular to a redshift, of the sensor stripes relative to the reference stripes, resulting in a contrast change between the sensor and reference stripes at the at least one of the (or the single) recording wavelength(s).

7. The method according to claim 6, wherein detecting the presence of the analyte comprises the observation of changes in light absorption at the at least one wavelength over the implant's length.

8. The method according to claim 6 or claim 7, wherein only one wavelength is used at a known signal frequency as a sensing wavelength, and a second wavelength is optional for the correction of changes unrelated to the presence of the analyte.

9. The method according to claim 8, wherein the sensing wavelength is λᵣₑₛ + *fwhm*/*2,* and the optional correction wavelength is λᵣₑₛ - *fwhm*/*2,* whereas λᵣₑₛ is the specific resonance wavelength for the employed nanoparticles and *fwhm* is the spectrum's full width at half maximum.

10. The method according to claim 9, wherein the sensing wavelength ranges between 600 and 1.000 nm, preferably 790 nm, and the optional correction wavelength ranges between 500 and 800 nm, preferably 690 nm.

11. The method according to any one of claims 7 to 10, wherein the detected changes are processed using a Fourier transformation, in particular a discrete cosine transformation, to identify and quantify the analyte based on modulation of the contrast at the spatial frequency corresponding to the spacing of sensor and reference stripes or a multiple thereof.

12. The method according to any one of claims 6 to 11, wherein the sensor is illuminated from the side of the implant, preferable with a light guide in contact with the skin, preferably from both sides of the implant, preferably with light emitted from an LED or laser and wherein the reflected light emitted from the area of the skin with the implant is recorded with a recording device, preferably a camera, line detector or photo-detector.

13. The method according to claim 12, wherein the illumination is monochromatic at a given time, or if more than one wavelength is recorded, the illumination switches between different monochromatic light sources (LED, lasers).

14. The method according to claim 12, wherein the illumination is broad ("white") and a filter in front of the recording camera is used to select one wavelength at a given time, or if more than one wavelength is desired to be recorded, the filter changes over time.

15. The method according to claim 12, wherein more than one wavelength is recorded by using two or more cameras simultaneously with filters or dichroic mirrors in front of them.

16. A method for manufacturing a sensor skin implant, in particular a skin sensor implant according to any one of claims 1 to 5, comprising the steps of:
- providing a bio-integrable macroporous hydrogel,
- embedding a plurality of alternating reference stripes and sensor stripes that are arranged next to each other to form a repetition unit, wherein the plurality of stripes is equipped with one type of nanoparticles, wherein the nanoparticles in the reference stripes are of a type that do not interact with the analyte, whereas the nanoparticles of the sensor stripes are coupled to or coated with a receptor bearing specific binding sites for an analyte.

17. The method according to claim 16, wherein the bio-integrable macroporous hydrogel with the plurality of alternating reference stripes and sensor stripes are fabricated by
a. filling polymethyl methacrylate (PMMA) beads in a suitable form and sintering the beads together to form a beadcake,
b. presoaking a first piece of beadcake in polymer solution and placing it in a polymerization mold,
c. filling up the polymerization mold with polymer solution to fully cover the beadcake,
d. stacking another presoaked beadcake on the first piece of beadcake,
e. repeating the steps a. to d. using polymer solution containing sensor and reference nanoparticles alternatingly to give a plurality of stocked beadcakes,
f. ablating the resulting block of stocked beadcakes to give access to the inner porous structure,
g. dissolving the beadcakes in diclormethane (DCM) to give a macroporous polymer,
h. cutting the polymer into suitable pieces, giving multiple implants from the same block.

## Amended claims

### Amended claims in accordance with Rule 137(2) EPC.

1. A sensor skin implant, comprising a plurality of alternating reference stripes and sensor stripes that are arranged next to each other to form a repetition unit, wherein the sensor stripes and references stripes comprise a bio-integrable macroporous hydrogel and are equipped with one type of nanoparticles, wherein the nanoparticles of the sensor stripes are coupled to or coated with a receptor bearing specific binding sites for an analyte, wherein the sensor skin implant is configured for detecting the presence of an analyte by recording reflection spectra at least one wavelength in the near-infrared regime, wherein a binding of the analyte leads to a shift of the sensor stripes relative to the reference stripes resulting in a contrast change between the sensor and reference stripes, and wherein the detection comprises observing changes in light absorption at the at least one wavelength over the implant's length, **characterized in that** the detected changes are processed using a Fourier transformation to identify and quantify the analyte based on modulation of the contrast at the spatial frequency corresponding to the spacing of sensor and reference stripes or a multiple thereof.

2. The sensor skin implant according to claim 1, wherein the bio-integrable macroporous hydrogel comprises a copolymer of poly(2-hydroxyethyl methacrylate) and poly(ethylene glycole)diacrylate.

3. The sensor skin implant according to any one of claims 1 to 2, wherein the sensor stripes and/or the reference stripes have each a length in the range between 2.0 and 4.0 mm, and a width in the range between 1.0 and 2.0 mm, preferably a width of 1.6 mm.

4. The sensor skin implant according to any one of claims 1 to 3, wherein the sensor stripes are equipped with plasmonic nanoparticles, preferably gold or gold alloy nanoparticles, that have a resonance wavelength in the near-infrared regime, preferably ranging from 700 nm to 3.000 nm, particularly preferably between 700 nm to 800 nm.

5. The sensor skin implant according to claim 4, wherein the plasmonic gold nanoparticles are coated with an aptamer as receptor that specifically binds to the analyte, wherein the analyte is a biomarker indicative for the medical indication, preferably kanamycin, glucose, insulin, oxygen, or a cancer marker, or hormone, or inflammation marker.

6. Use of a sensor skin implant according to any one of claims 1 to 5 for detecting the presence of an analyte.

7. A method for detecting the presence of an analyte, by
detecting the presence of analyte bound to a receptor of a sensor skin implant according to any one of claims 1 to 5, comprising the steps of recording reflection spectra atat least one wavelength in the near-infrared regime, the wavelength preferably being in the range from 700 nm to 3.000 nm, particularly preferably between 700 nm to 800 nm, wherein a binding of the analyte leads to a shift, in particular to a redshift, of the sensor stripes relative to the reference stripes, resulting in a contrast change between the sensor and reference stripes at the at least one of the (or the single) recording wavelength(s), wherein the detection comprises observing changes in light absorption at the at least one wavelength over the implant's length, **characterized in that** the detected changes are processed using a Fourier transformation to identify and quantify the analyte based on modulation of the contrast at the spatial frequency corresponding to the spacing of sensor and reference stripes or a multiple thereof.

8. The method according to claim 7, wherein detecting the presence of the analyte comprises the observation of changes in light absorption at the at least one wavelength over the implant's length.

9. The method according to claim 7 or claim 8, wherein only one wavelength is used at a known signal frequency as a sensing wavelength, and a second wavelength is optional for the correction of changes unrelated to the presence of the analyte.

10. The method according to claim 9, wherein the sensing wavelength is λᵣₑₛ + *fwhm*/*2,* and the optional correction wavelength is λᵣₑₛ - *fwhm*/*2,* whereas λᵣₑₛ is the specific resonance wavelength for the employed nanoparticles and *fwhm* is the spectrum's full width at half maximum.

11. The method according to claim 10, wherein the sensing wavelength ranges between 600 and 1.000 nm, preferably 790 nm, and the optional correction wavelength ranges between 500 and 800 nm, preferably 690 nm.

12. The method according to any one of claims 7 to 11, wherein the Fourier transformation is a discrete cosine transformation.

13. The method according to any one of claims 7 to 12, wherein the sensor is illuminated from the side of the implant, preferable with a light guide in contact with the skin, preferably from both sides of the implant, preferably with light emitted from an LED or laser and wherein the reflected light emitted from the area of the skin with the implant is recorded with a recording device, preferably a camera, line detector or photodetector.

14. The method according to claim 13, wherein the illumination is monochromatic at a given time, or if more than one wavelength is recorded, the illumination switches between different monochromatic light sources (LED, lasers).

15. The method according to claim 13, wherein the illumination is broad ("white") and a filter in front of the recording camera is used to select one wavelength at a given time, or if more than one wavelength is desired to be recorded, the filter changes over time.

16. The method according to claim 13, wherein more than one wavelength is recorded by using two or more cameras simultaneously with filters or dichroic mirrors in front of them.

17. A method for manufacturing a sensor skin implant, in particular a skin sensor implant according to any one of claims 1 to 5, comprising the steps of:
- providing a bio-integrable macroporous hydrogel,
- embedding a plurality of alternating reference stripes and sensor stripes that are arranged next to each other to form a repetition unit, wherein the plurality of stripes is equipped with one type of nanoparticles, wherein the nanoparticles in the reference stripes are of a type that do not interact with the analyte, whereas the nanoparticles of the sensor stripes are coupled to or coated with a receptor bearing specific binding sites for an analyte.

18. The method according to claim 17, wherein the bio-integrable macroporous hydrogel with the plurality of alternating reference stripes and sensor stripes are fabricated by
a. filling polymethyl methacrylate (PMMA) beads in a suitable form and sintering the beads together to form a beadcake,
b. presoaking a first piece of beadcake in polymer solution and placing it in a polymerization mold,
c. filling up the polymerization mold with polymer solution to fully cover the beadcake,
d. stacking another presoaked beadcake on the first piece of beadcake,
e. repeating the steps a. to d. using polymer solution containing sensor and reference nanoparticles alternatingly to give a plurality of stocked beadcakes,
f. ablating the resulting block of stocked beadcakes to give access to the inner porous structure,
g. dissolving the beadcakes in diclormethane (DCM) to give a marcoporous polymer,
h. cutting the polymer into suitable pieces, giving multiple implants from the same block.
